# EUROPEAN PATENT SPECIFICATION

(11) **EP 1 381 345 B1**
(45) Date of publication and mention of the grant of the patent: **09.07.2008**
(21) Application number: 02725276.6
(22) Date of filing: 22.03.2002
(51) Int. Cl.: A23K 1/00, A23K 1/18

(54) **DELIVERY OF DISEASE CONTROL IN AQUACULTURE USING YEASTS CONTAINING BIOACTIVE PROTEINS**
ABGABE VON MITTELN ZUR KRANKHEITSKONTROLLE IN AQUAKULTUR UNTER VERWENDUNG VON BIOAKTIVE PROTEINE ENTHALTENDER HEFE
ADMINISTRATION DE TRAITEMENT CONTRE UNE MALADIE EN AQUACULTURE AU MOYEN DE LEVURES CONTENANT DES PROTEINES BIO-ACTIVES

(30) Priority: 23.03.2001 US 277947 P
(43) Date of publication of application: 21.01.2004
(60) Divisional application: 04030873.6
(73) Proprietor: Advanced Bionutrition Corporation, Columbia, MD 21045 (US)
(72) Inventor: KYLE, David, J., Catonsville, MD 21228 (US)
(74) Representative: Ahner, Francis
(86) International application number: PCT/US2002/008651
(87) International publication number: WO 2002/076391

(56) References cited:
- EP-A- 0 239 075
- WO-A-02/03812
- WO-A-02/38770
- WO-A-99/67398
- WO-A1-90/07578
- WO-A1-95/35389
- WO-A2-97/14806
- GB-A- 2 316 082
- US-A- 5 661 017
- US-A- 6 100 388
- US-A1- 5 681 557
- US-A1- 2002 090 376
- US-A1- 2002 102 692
- US-B1- 6 399 074
- US-B2- 6 462 027
- TUSE DANIEL: 'Single-cell protein: current status and furure prospects' CRITICAL REVIEWS IN FOOD SCIENCE AND NUTRITION vol. 19, no. 4, 1984, pages 273 - 325, XP002959284
- LEONG ET AL.: 'Fish vaccine antigens produced or delivered by recombinant DNA technologies' DEVELOPMENTS IN BIOLOGICAL STANDARDIZATION vol. 90, 1997, pages 267 - 277, XP001062656
- GRANT ET AL.: 'Expression of yellow tail (seriola quinqueradiata) fish growth hormone cDNA in the marine photosynthetic bacterium rhodobacter SP NKPB 0021' BIOTECHNOLOGY LETTERS vol. 15, no. 2, February 1993, pages 111 - 114, XP002959285
- SETO ET AL.: 'Production of eicosapentaenoic acid by a marine microalgae and ITs commercial utilization for aquaculture' IND. APP. SINGLE CELL OILS 1992, pages 219 - 234, XP002959286

## Description

### BACKGROUND OF THE INVENTION

### Field of the Invention

This invention is directed to the use of yeast cells which are used as feed components in aquaculture, and which also contain exogenous peptides and/or antibodies, which will convey resistance or immunity to viral or bacterial pathogens or otherwise improve the health and performance of the species consuming said microbial cells. The peptides and/or antibodies may be expressed inside the microbial cells by direct genetic modification of the microbe itself, or by the infection of the microbe with a virus that has been altered to express the protein of interest.

### Related Art

Certain plant products have been produced using specific genetic modification to express proteins and/or antibodies of therapeutic value. The group at the Boyce Thompson Institute at Cornell has been cloning viral coat protein into bananas and potatoes so that when ingested, this will be equivalent to delivering an oral vaccine. This concept has not been extended to microbes.

There are several plant biotech companies such as Meristem, Large Scale Biology, and Prodigene, which are now expressing certain human therapeutic proteins in the plants including antibodies.

Recombinant microbes Including bacteria, yeast and fungi have been used to produce human therapeutic proteins. However, such recombinant microbes have not been used for agricultural purposes incorporating ingestion of the whole organism. In both the plant and microbial cases, the recombinant organism has simply been used as a factory, and the therapeutic protein is then isolated and purified prior to use.

Certain plant products have been produced which contain proteins and/or antibodies of therapeutic value by infecting the plant with a virus that expresses the protein of interest. Large Scale Biology has a series of patents protecting this technology but these patents do not disclose the use of the technology in microbes and certainly not algae.

Antibiotic doping is used routinely in the aquaculture setting. Typically, the pure or semipure antibiotics are added directly to the water column; however, crude fermentation broths, or crude preparations including cells, have not been used for any kind of therapeutic delivery system.

Production of amino acids such as lysine typically involves a genetically modified microorganism, which overproduces the amino acid of interest and excretes it into the fermentation medium. The wastestream from such a fermentation would include biomass containing the amino acid, and this wastestream product could be used as a crude delivery form of the small molecule nutritive amino acid.
LEONG et al. (Dev. Biol. Stand., vol. 90, p:267-277, 1997) relates to fish vaccine antigens produced or delivered by recombinant DNA technologies. This document only envisages yeast as a host system for the production of viral antigen, said viral antigen being finally purified from said host system before its injection to fish.
US 5,681,557 relates to methods of increasing monocyte cytotoxicity directed against microbial infections with Interleukin-7. This document only envisages yeast as a host system for the production of Interleukin-7, said Interleukin-7 being orally administrated after its purification.
WO 90/07578 relates to recombinant fish growth hormones exhibiting growth stimulation and osmoregulatory effects. This document only envisages yeast as a host system for the production of said growth hormones, said growth hormones being incorporated into fish feed after their recovering from the yeast culture medium.
WO 02/38770 relates to vaccine compositions to protect fish against infectious pancreatic necrosis virus, but does not disclose the use of a yeast biomass infected with a recombinant virus expressing antibodies or bactericidal peptides for the production of a feed nor the use of such a yeast biomass for the preparation of a medicament.

### SUMMARY OF THE INVENTION

The present invention provides for a process for the production of a feed and the use of this feed for the delivery of a therapeutic dose of a bioactive peptide or protein.

In one embodiment, this invention provides a process for the production of a feed composition in aquaculture comprising the steps of:
a) infecting a yeast biomass with a recombinant virus expressing antibodies or bactericidal and bacteriostatic peptides selected in the group comprising cecropins, panaedins, bactenecins, callinectins, myticins, tachyplesins, clavanins, misugrins, pleurocidins, parasins, histones, acidic proteins, and lysozymes and which are non native to said yeast biomass,
b) culturing yeast biomass obtained in step a) to produce said antibodies bactericidal and bacteriostatic peptides, and
c) formulating yeast biomass obtained in step b) as a feed composition.

In another embodiment, this invention provides a method of preparing a feed for delivering therapeutic proteins to an animal, said feed comprising a yeast expressing a non-native therapeutic protein. This method is particularly suitable for fish or shellfish in aquaculture. In a preferred mode, the therapeutic protein is a recombinant protein expressed by the yeast or the yeast is infected by a recombinant virus, which expresses the recombinant therapeutic or bioactive protein. Preferred therapeutic proteins include a protein which inhibits Taura Syndrome Virus (TSV) or White Spot Syndrome Virus (WSSV) infection in shrimp.

The marine environment is filled with bacteria and viruses that can attack fish and shellfish thereby devastating intensive farms very quickly. Bacteria and viruses can also attack single celled microalgae, so these organisms have evolved biochemical mechanisms to defend themselves from such attacks. Such mechanisms may involve the secretion of compounds that inhibit bacterial growth or viral attachment. Such compounds are called "prebiotics" and have effects similar to how cranberry juice can prevent bladder infections in humans. When nutritional, therapeutic or protective effects are delivered via the whole live organisms, such as *Lactobacillis* in yogurt, such products are referred to as "probiotics" and the organisms are "probionts". For the purposes of this invention, both types of action will be referred to as probiotic.

The main aspect of this invention is directed to the use of recombinant virus infected yeast to deliver the bioactive protein of choice. The recombinant virus-infected yeast may be tested for antibiotic activity by standard antibiotic screening assays to confirm their activity.

Historically, only bacteria have been used in a probiotic fashion to alter a pond's ecology in order to eliminate or reduce the number of pathogenic bacteria. A problem with the bacterial probiotic approach is that the existing microbial ecology represents a massive buffer that is difficult to modulate with the introduction of relatively small numbers of alternative bacteria and the results to date have been unimpressive. Furthermore, even if the newly introduced bacteria do bloom, any large increase in bacterial levels in a pond can lower oxygen levels and cause harm to the fish or shrimp. The use of photosynthetic microalgae overcomes this problem as they actually increase oxygen levels. Microalgae have not been considered before as probiotics. Previous experience in the screening of extensive algal culture collections has indicated a number of algal species that exhibit antibacterial or bacteriostatic capabilities. Some of these activities may be anti-Vibrio activity. Such species would be candidates for a high value enrichment feed that delivers both nutritional and antibiotic capabilities. This invention provides an approach to disease control which may be the solution to an impending ecological disaster that will result from the present uncontrolled practice of dumping of toxic chemicals and antibiotics into the water systems to control these bacterial, fungal or viral pathogens.

One of the major disease control problems in shrimp aquaculture today is infection by certain viruses (e.g., White Spot Syndrome Virus and Tara Syndrome Virus). Neither current antibiotic, nor probiotic strategies will work in this situation, and shrimp cannot be vaccinated in a way similar to fish. Shrimp have only a rudimentary immune system so they are particularly susceptible to devastation by viral attacks. This invention provides a solution to this problem using a biological control method using yeast as the vector to deliver anti-White Spot antibodies directly to the shrimp. These "Designer feeds" would be a normal part of the diet, but modified to deliver a therapeutic dose of antibody directly to the gut of the shrimp. This approach is known as "passive immunity" because the antibody remains outside the host organism and simply prevents infestation through the gut wall. Such probiotics, as envisioned in the invention, do not have to replicate in the target organism for the desired effect to occur. Alternatively, the yeast itself may be infected with a virus that is engineered to produce the antibody of interest.

Antibodies to desired targets, such as White Spot Syndrome Virus or Taura Syndrome Virus, may be prepared by routine immunization and selection of monoclonal antibody producing hybridomas, or by screening viral or bacterial expression libraries of immunoglobulin genes and gene fragments. See "Current Protocols in Immunology," Coligan, et al., eds, Wiley Interscience, 1991, and periodic supplements. Nucleic acid sequences encoding the binding sites of the selected antibodies can be cloned using standard methods (see "Current Protocols in Molecular Biology." Ausubel, et al., eds., Wiley-Interscience, 1987, and periodic supplements), and antibodies may be expressed from recombinant microbes (including algae, see, e.g., U.S. Patent No. 6,027,900) or cloned into viruses that infect the desired microbes.

There are a number of bactericidal and bacteriostatic peptides, which will inhibit microbial growth and that include, but are not limited to cecropins, penaeidins, bactenecins, callinectins, myticins, tachyplesins, clavanins, misgurins, pleurocidins, parasins, histones, acidic proteins, and lysozymes. These peptides may be expressed in a yeast biomass using recombinant methods as described above, and thus provided as a feed component to convey resistance to infestation.

The invention as contemplated herein, is described in the following examples, but its utility is not limited to the examples provided.

### EXAMPLES

**Example 1 incorporation of an antibody into yeast feed.** A particular viral or bacterial pathogen is chosen and used to prepare monoclonal antibodies using procedures well known to experts in this field. Gene(s) coding for this antibody or an appropriate antibody fragment (Fab or Fv) are isolated and amplified in the appropriate vector. The gene is spliced into a transformation vector suitable for a yeast (e.g. *Saccharomyces*). The transformation vector is chosen so that the antibody will be over expressed in the microbial cell biomass. This biomass is then used as a feed additive in such a way as to provide the antibody directly to the animal thus providing passive immunity.

**Example 2 Expression of a bactericidal protein in a microbial feed.** A bactericidal protein is chosen for the particular application. For example, proteins of the penaeidin class may be chosen for pathogenic control in shrimp. Penaeidins are members of a family of antimicrobial peptides isolated from crustaceans (e.g., Penaeid shrimp). Antimicrobial peptides may also come from insects and chelicerates and may include but are not limited to cecropins, peneaidins, bactenecins, callinectins, myticins, tachyplesins, clavanins, misgunins, pleurocidins, parasins, histones, acidic proteins, and lysozymes. The gene for the chosen protein or peptide is either isolated from the original source, an amplification source, or it can be made synthetically. The gene is then incorporated into a transformation vector suitable for a yeast (e.g. *Saccharomyces*). The transformation vector is chosen so that the protein will be over expressed in the microbial cell biomass. This biomass is then used as a feed additive in such a way as to provide the bactericidal protein directly to the animal thus providing resistance to that particular pathogen.

## Claims

1. Process for the production of a feed composition in aquaculture comprising the steps of:
a) infecting a yeast biomass with a recombinant virus expressing antibodies,
b) culturing yeast biomass obtained in step a) to produce said antibodies, and
c) formulating yeast biomass obtained in step b) as a feed composition.

2. Process according to claim 1 for shrimp aquaculture.

3. Process according to claim 1, wherein said antibodies target White Spot Syndrome virus or Taura Syndrome virus.

4. Process for the production of a feed composition in aquaculture comprising the steps of:
a) infecting a yeast biomass with a recombinant virus expressing bactericidal and bacteriostatic peptides selected in the group comprising cecropins, panaedins, bactenecins, callinectins, myticins, tachyplesins, clavanins, misugrins, pleurocidins, parasins, histones, acidic proteins, and lysozymes, and which are non native to said yeast biomass,
b) culturing yeast biomass obtained in step a) to produce said expressing bactericidal and bacteriostatic peptides, and
c) formulating yeast biomass obtained in step b) as a feed composition.

5. Process according to claim 4 for fish or shell fish.

6. Process according to claim 4 for shrimp.

7. Use of a feed composition in aquaculture obtainable by the process according to claim 3 for the preparation of a medicament which inhibits Taura Syndrome Virus (TSV) or White Spot Syndrome Virus (WSSV) infection in shrimp.

8. Use of a feed composition in aquaculture obtainable by the process according to claim 4 for the preparation of a medicament that conveys resistance to infestation.

## Patentansprüche

1. Verfahren zur Herstellung einer Futtermittelzusammensetzung in Aquakultur, umfassend die Schritte:
a) Infizieren einer Hefe-Biomasse mit einem rekombinanten Virus, der Antikörper exprimiert,
b) Kultivieren der in Schritt a) erhaltenen Hefe-Biomasse, um die Antikörper zu produzieren, und
c) Formulieren der in Schritt b) erhaltenen Hefe-Biomasse als Futtermittelzusammensetzung.

2. Verfahren nach Anspruch 1 für Krabben-Aquakultur.

3. Verfahren nach Anspruch 1, bei dem die Antikörper gegen den White-Spot-Syndrom-Virus oder Taura-Syndrom-Virus gerichtet sind.

4. Verfahren zur Herstellung einer Futtermittelzusammensetzung in Aquakultur, umfassend die Schritte:
a) Infizieren einer Hefe-Biomasse mit einem rekombinanten Virus, der bakterizide und bakteriostatische Peptide exprimiert, die ausgewählt sind aus der Gruppe umfassend Cecropine, Panaedine, Bactenecine, Callinectine, Myticine, Tachyplesine, Clavanine, Misugrine, Pleurocidine, Parasine, Histone, saure Proteine und Lysozyme und die für die Hefe-Biomasse nicht nativ sind,
b) Kultivieren der in Schritt a) erhaltenen Hefe-Biomasse, um die exprimierenden bakteriziden und bakteriostatischen Peptide zu produzieren, und
c) Formulieren der in Schritt b) erhaltenen Hefe-Biomasse als Futtermittelzusammensetzung.

5. Verfahren nach Anspruch 4 für Fisch oder Schalentiere.

6. Verfahren nach Anspruch 4 für Krabben.

7. Verwendung einer Futtermittelzusammensetzung in Aquakultur, erhältlich durch das Verfahren nach Anspruch 3, für die Herstellung eines Medikaments, das Taura-Syndrom-Virus (TSV) oder White-Spot-Syndrom-Virus (WSSV)-Infektion bei Krabben hemmt.

8. Verwendung einer Futtermittelzusammensetzung in Aquakultur, erhältlich durch das Verfahren nach Anspruch 4, für die Herstellung eines Medikaments, das eine Resistenz gegen einen Befall vermittelt.

## Revendications

1. Procédé de production d'une composition alimentaire en aquaculture, qui comprend les étapes :
a) d'infection d'une biomasse de levure avec un virus recombinant exprimant des anticorps,
b) de culture de la biomasse de levure obtenue dans l'étape a) pour produire lesdits anticorps, et
c) de formulation de la biomasse de levure obtenue dans l'étape b) en une composition alimentaire.

2. Procédé selon la revendication 1, pour l'aquaculture de crevettes.

3. Procédé selon la revendication 1, dans lequel lesdits anticorps ciblent le virus de la maladie des points blancs ou le virus du syndrome de Taura.

4. Procédé de production d'une composition alimentaire en aquaculture, qui comprend les étapes :
a) d'infection d'une biomasse de levure avec un virus recombinant exprimant des peptides bactéricides et bactériostatiques choisis dans le groupe comprenant les cécropines, les penaeidines, les bacténécines, les callinectines, les myticines, les tachyplésines, les clavanines, les misgurines, les pleurocidines, les parasines, les histones, les protéines acides, et les lysozymes, et qui ne sont pas natifs de ladite biomasse de levure,
b) de culture de la biomasse de levure obtenue dans l'étape a) pour produire lesdits peptides d'expression bactéricides et bactériostatiques, et
c) de formulation de la biomasse de levure obtenue dans l'étape b) en une composition alimentaire.

5. Procédé selon la revendication 4, pour les poissons ou les coquillages et crustacés.

6. Procédé selon la revendication 4, pour les crevettes.

7. Utilisation d'une composition alimentaire en aquaculture, qui peut être obtenue par le procédé selon la revendication 3, pour la préparation d'un médicament qui inhibe une infection par le virus du syndrome de Taura (TSV) ou par le virus de la maladie des points blancs (WSSV) chez les crevettes.

8. Utilisation d'une composition alimentaire en aquaculture, qui peut être obtenue par le procédé selon la revendication 4, pour la préparation d'un médicament qui confère la résistance aux infestations.
